# FASCICULE DE BREVET EUROPEEN

(11) **EP 1 651 287 B1**
(45) Date de publication et mention de la délivrance du brevet: **09.01.2008**
(21) Numéro de dépôt: 04767601.0
(22) Date de dépôt: 07.07.2004
(51) Int. Cl.: A61L 27/12, A61P 35/00, A61K 49/08

(54) **COMPOSITE INJECTABLE POUR MAGNETOCYTOLYSE DE CELLULES METASTATIQUES OSSEUSES**
INJIZIERBARER KOMPOSIT FÜR DIE MAGNETOZYTOLYSE VON KNOCHENMETASTASEZELLEN
INJECTABLE COMPOSITE FOR THE MAGNETOCYTOLYSIS OF BONE METASTATIC CELLS

(30) Priorité: 08.07.2003 FR 0308332
(43) Date de publication de la demande: 03.05.2006
(73) Titulaire: Urodelia, 31470 Saiguede (FR)
(72) Inventeur: FRAYSSINET, Patrick, Pierre, F-31470 Saiguede (FR); ROUQUET, Nicole, Francine, F-31400 Toulouse (FR)
(74) Mandataire: Warcoin, Jacques
(86) Numéro de dépôt international: PCT/FR2004/001766
(87) Numéro de publication internationale: WO 2005/004942

(56) Documents cités:
- EP-A- 0 361 797
- EP-A- 0 928 611
- US-A1- 2003 077 225
- US-B1- 6 565 887
- DATABASE WPI Section Ch, Week 199205 Derwent Publications Ltd., London, GB; Class L03, AN 1992-035168 XP002270390 & JP 03 275522 A (NOK CORP) 6 décembre 1991 (1991-12-06)
- HAFELI U O ET AL: "Effective Targeting of Magnetic Radioactive <90>Y-microspheres to Tumor Cells by an Externally Applied Magnetic Field. Preliminary In Vitro and In Vivo Results" NUCLEAR MEDICINE AND BIOLOGY, ELSEVIER SCIENCE PUBLISHERS, NEW YORK, NY, US, vol. 22, no. 2, 1 février 1995 (1995-02-01), pages 147-155, XP004051845 ISSN: 0969-8051
- EBISAWA Y ET AL: "Bioactivity of ferrimagnetic glass-ceramics in the system FeO-Fe2O3-CaO-SiO2" BIOMATERIALS, ELSEVIER SCIENCE PUBLISHERS BV., BARKING, GB, vol. 18, no. 19, 1997, pages 1277-1284, XP004088021 ISSN: 0142-9612

## Description

La présente invention se rapporte à un matériau dégradable biocompatible composé d'une matrice phosphocalcique comprenant des particules magnétiques, ledit matériau étant utile pour le traitement des métastases osseuses par thermolyse et/ou pour le traçage des cellules cancéreuses.

Les métastases osseuses de cancers primaires situés ailleurs dans l'organisme sont très fréquentes, elles sont même une évolution attendue de la maladie au fur et à mesure que l'espérance de vie des patients augmente. Le tissu osseux est une des trois localisations les plus fréquentes des métastases cancéreuses. Elles sont particulièrement fréquentes dans l'histoire naturelle des cancers du sein, de la prostate, du poumon, du rein et de la thyroïde. Elles constituent, radiologiquement dans la majorité des cas, une lacune en raison de la forte activité ostéolytique qui se manifeste dans leur périphérie. Cette ostéolyse s'accompagne cliniquement de douleurs osseuses et de fractures d'os longs ou tassements de corps vertébraux. Le pronostic des métastases osseuses reste très sombre et leur traitement est palliatif. Des survies prolongées peuvent néanmoins être obtenues en fonction des caractéristiques de la tumeur primaire.

Histologiquement, les métastases sont classées en tumeurs ostéolytiques, ostéoblastiques mixtes et de type intertrabéculaires. Le type le plus fréquent est le type mixte dans lequel une zone de résorption osseuse coexiste avec une zone de régénération en périphérie de la première. Les métastases sont constituées d'amas cellulaires entre les trabécules ou les restes de trabécules. Au niveau cellulaire, il existe un grand polymorphisme et de nombreuses cellules atypiques avec de nombreuses images de mitoses. Elles sont entourées de tissu osseux montrant de nombreux signes de résorption sous la forme de nombreuses lacunes de Howship avec des ostéoclastes actifs.

Les cellules constituant ces amas cellulaires proviennent de la migration de cellules de la tumeur primaire qui se fixent dans le tissu osseux car elles trouvent des conditions favorables à leur développement. Les métastases osseuses peuvent être également classées en différents stades de développement : phase d'apparition, phase d'interaction, et phase carcinomateuse. Elles sont le plus souvent diagnostiquées pendant la phase d'interaction c'est-à-dire lorsque les cellules tumorales activent la formation et l'activité d'ostéoclastes qui sont les cellules chargées de la résorption osseuses. Il semble que les cellules tumorales collaborent avec les cellules stromales et les ostéoblastes pour le recrutement d'ostéoclastes à travers le système RANK-RANKL (Kitazawa, S., et Kitazawa,R., RANK ligand is a prerequisite for cancer-associated osteolytic lesions, J Pathol, 2002 : 198 ; 228-236).

L'objectif de l'invention est le traitement des métastases osseuses. Il permet d'éliminer les cellules cancéreuses pour limiter la progression de la maladie et pour abolir la stimulation des ostéoclastes sans, autant que faire se peut, détruire les capacités de régénération osseuse en périphérie de la tumeur. L'élimination des cellules cancéreuses aboutit cliniquement à une diminution de la douleur et une baisse du risque fracturaire.

Plusieurs types de traitements sont habituellement associés pour réduire le volume tumoral. La radiothérapie externe est habituelle, souvent associée à un traitement chimiothérapique. L'échappement à la chimiothérapie est relativement précoce et les risques hématologiques ainsi que l'inconfort inhérents à la chimiothérapie par voie générale restent un problème majeur de ce type de traitement. La radiothérapie n'est pas dénuée de risque, en particulier, lorsque la métastase est située à proximité d'un organe noble, poumon, système nerveux central... D'autre part, le problème de ces traitements, lorsqu'ils sont efficaces, provient de l'élimination concomitante des cellules impliquées dans la régénération osseuse.

D'autres traitements actifs au niveau de la métastase, peuvent être mis en oeuvre à visée antalgique. L'injection intraveineuse d'isotope du strontium ainsi que l'utilisation de biphosphonates s'est révélée efficace. Ces composés ne conduisent pas ou peu à l'élimination des cellules cancéreuses.
Des voies de traitement originales sont actuellement développées afin d'avoir une action plus spécifique sur la cellule cancéreuse. Elles sont destinées à éviter les effets secondaires des chimio et radiothérapies qui entraînent la cytolyse des cellules à prolifération rapide et sont donc toxiques sur les cellules souches sanguines et celles des épithéliums à renouvellement rapide. Elles sont basées sur le principe du transport d'une molécule inactive au contact des cellules tumorales, la molécule étant activées quand elle a atteint la tumeur. La fixation de molécules cytotoxiques sur des particules magnétiques que l'on va injecter par voie intraveineuse et que l'on concentre dans la tumeur grâce à un champ magnétique ou à des molécules organiques spécifiques des récepteurs des cellules tumorales est une méthode qui a suscité de nombreuses recherches (Yanase, M., Shinkai,M., Honda, H., Wakabayashi, T., Yoshida, J., Kobayashi, T., intracellular hyperthermia for cancer using magnetite cationic liposomes : an in vivo study, Jpn J Cancer Res ; 89 (4) : 463-9, 1998 ; Moroz, P., Jones, S., Gray, B.N., Magnetically mediated hyperthermia : current status and future directions, Int J Hyperthermia ; 18 : 267-84, 2002 ; Pulfer, S.K., Gallo, J.M., Targeting magnetic microsphere to brain tumors, in: Häfeli, U., Schütt, W., Teller, J., Zborowski, M., (eds) Scientific and clinical applications of magnetic carriers. Plenum Press New York 1997, pp 445-456). Les particules une fois phagocytées ou internalisées par les cellules peuvent avoir une toxicité propre grâce au principe actif qu'elles véhiculent et qu'elles relarguent dans la cellule. Elles peuvent également être chauffées dans un champ magnétique à haute fréquence et induire une thermolyse directe ou bien indirectement par sensibilisation des cellules chauffées à la radio ou la chimiothérapie.

Les obstacles majeurs à ce type de thérapie proviennent de la spécificité plus ou moins bonne de l'adressage des particules. En fonction de leurs caractéristiques de surface et des protéines plasmatiques se fixant à leur surface, elles vont être ou pas éliminées par phagocytose dans la rate et le foie par des macrophages avant qu'elles ne puissent être activées au contact des cellules cancéreuses (Müller, R.H., Lück, M., Harnisch,S., Thode, K., Intravenously injected particles : surface properties and interaction with blood proteins - The key determining the organ distribution. in: Häfeli, U., Schütt, W., Teller, J., Zborowski, M., (eds) Scientific and clinical applications of magnetic carriers. Plenum Press New York 1997, pp 135-148). Un autre obstacle est lié à la plus ou moins grande spécificité des molécules chargées de la reconnaissance cellulaire ainsi que la possibilité, pour les particules, de passer la barrière vasculaire.

La possibilité d'injecter, directement dans la tumeur, les microparticules en suspension dans un liquide a également été évoquée. Cependant, l'injection est délicate car il est difficile dans ces conditions de savoir où vont les particules. Les particules doivent pouvoir pénétrer dans les tumeurs puis dans les cellules qu'elles doivent détruire. L'efficacité des particules dans la thermocytolyse est liée à leur contact avec les cellules. Les particules non liées aux cellules sont beaucoup moins efficaces (Bacri, J.C., de Fatima Da Silva, M., Perzynski, R., Pons, J.N., Roger, J., Sabolovic, D., Halbreich, A., Use of magnetic nanoparticles for thermolysis of cells in a ferrofluid.. in: Häfeli, U., Schütt, W., Teller, J., Zborowski, M., (eds) Scientific and clinical applications of magnetic carriers. Plenum Press New York 1997, pp 597-606). Les cellules du système des phagocytes mononucléés présentes dans le sang et de nombreux tissus sont capables de phagocyter des particules jusqu'à une taille d'environ une quarantaine de µm. Les autres cellules ont des capacités de phagocytose beaucoup plus limitées. En revanche, quasiment toutes les cellules sont capables d'ingérer par endocytose des particules de taille beaucoup plus réduites. Il est généralement considéré que les particules de taille inférieures à 100 nm sont capables de passer la membrane cellulaire par endocytose. Il suffit pour cela que les cellules soient au contact des particules.

EP 0361797 décrit un matériau pour le traitement par hyperthermie des tumeurs osseuses, comprenant des particules ferromagnétiques dans une matrice céramique non biodégradable.

Ainsi, il apparaît nécessaire de trouver des solutions thérapeutiques alternatives pour répondre aux problèmes mentionnés ci-dessus concernant l'utilisation des particules magnétiques en cancérologie.

Dans le cadre de l'invention, nous avons développé un système permettant d'injecter dans une tumeur une pâte contenant une suspension minérale à partir de laquelle va précipiter un sulfate ou un phosphate de calcium contenant des particules magnétiques de petites tailles dispersées dans la matrice minérale formée qui vont être libérées au fur et à mesure de la dégradation de celle-ci. Le rôle de la matrice est de contenir les particules dans la zone d'injection, les maintenir séparées les unes des autres et les libérer au contact des cellules tumorales suivant une cinétique définie. Les particules libérées vont ensuite pénétrer dans les cellules tumorales par endocytose.

Plusieurs avantages sont obtenus avec ce matériau : Les matrices de phosphates et de sulfates de calcium présentent une bonne biocompatibilité osseuse. Ils sont, pour la plupart, capables d'être totalement intégrés dans le tissu osseux sans provoquer de réaction à corps étranger importante. Ils sont ensuite dégradés à des vitesses variables suivant leur composition chimique et leurs caractéristiques physico-chimiques et totalement remplacés par le tissu osseux. Ces matrices sont injectés dans l'os sous forme d'une pâte d'où il précipite un composé de phosphate ou de sulfate de calcium différent de celui qui est en suspension dans la pâte. L'intrication des cristaux du précipité assure la prise du matériau et sa tenue mécanique qui peut être proche de celle de l'os spongieux en quelques heures à quelques jours (T. Yuasa, Y. Miyamato, K. Ishikawa, M. Takechi, M. Nagayama, and K. Suzuki. In vitro résorption of three apatite cements with osteoclasts. J Biomed Mater Res 54:344-350, 2001; S. Takagi, L. C. Chow, M. Markovic, C. D. Friedman, and P. D. Costantino. Morphological and phase characterizations of retrieved calcium phosphate cement implants. J Biomed Mater Res (Appl Biomater) 58:36-41, 2001 Y. Miyamato, T. Toh, T. Yuasa, M. Takechi, Y. Momota, M. Nagayama, K. Ishikawa, and K. Suzuki. Basic properties of apatite cement containing carbonate apatite and its résorption by cultured osteoclasts. In: Proceedings of the 13th Int Symp on Ceramics in Medicine,Anonymous Switzerland: 2001, p. 829-832.).

Par ailleurs, les particules magnétiques peuvent être chauffées dans un champ électromagnétique avant ou après avoir pénétré dans les cellules. Ce chauffage peut être répété le nombre de fois nécessaires sans nécessiter une autre injection. Les particules magnétiques sont, en effet, libérées sur plusieurs jours à plusieurs semaines à partir de la matrice minérale. Lorsque les cellules sont thermolysées, les particules qu'elles contenaient s'ajoutent à celles qui viennent d'être libérées de la matrice pour être phagocytées par de nouvelles cellules. De plus, ces particules agissant au coeur de la tumeur n'entravent pas la régénération osseuse existant dans la périphérie des métastases osseuses.

### Description

Ainsi, l'invention se rapporte à un matériau dégradable biocompatible caractérisé en ce qu'il est composé d'une matrice phosphocalcique et/ou de sulfate de calcium ou d'une matrice polymérique biocompatible dégradable, ladite matrice contenant des particules magnétiques, ledit matériau se trouvant sous la forme d'une pâte lors de l'introduction dans l'organisme et sous forme solide ultérieurement. Les particules sont libérées au fur et à mesure de la dégradation du ciment constituant la matrice dans laquelle elles sont emprisonnées.

Par "matrice phosphocalcique", on entend un mélange comprenant un ou plusieurs phosphates sélectionnés parmi le groupe des phosphates de calcium amorphe, des phosphates apatitiques faiblement cristallins, des phosphates dicalciques anhydres ou dihydratés, des phosphates tricalciques, des phosphates monocalciques monohydratés, des pyrophosphates, des phosphates octocalciques ou de l'hydroxyapatite. De préférence, la matrice phosphocalcique est rapidement résorbable, c'est à dire dans un délai de quelques jours à quelques semaines ce qui implique une solubilité correspondant à celle du phosphate dicalcique. Ladite matrice peut également être constituée tout ou partie de sulfates de calcium qui présentent des caractéristiques de biocompatibilité et de dégradation également compatibles avec les applications du matériau pour le traitement des tumeurs osseuses. Un tel matériau selon l'invention est un matériau minéral introduit sous forme de pâte. Lors de la prise, la précipitation d'une phase n'est pas dans la suspension ou bien est minoritaire dans cette dernière. Les premiers signes histologiques de résorption (encoches, fragmentation partielle) sont visibles au microscope quelques jours après son introduction, et la disparition radiologique survient entre 4-52 semaines suivant la composition de la phase minérale, préférentiellement 8 semaines pour les matrices de sulfates de calcium.

En outre, ledit matériau peut être composé d'une matrice polymérique dégradable contenant des particules magnétiques. Elle peut être constituée par un polymère biodégradable naturel ou artificiel, tels que collagène, acides polylactiques et glycoliques, polydioxanone, polyfumarate, plolyanhydres, polyorthoesters, polyurethanes, polyphosphazenes, polycaprolactone, polyhydroxybutyrate, polyhydroxyvalerate, polyvalerolactone, acide polytartronique et polymalonique. Le matériau peut-être sous forme de gel, de mousse ou de pâte.

Par "particules magnétiques", on entend des particules contenant un métal, notamment du fer, de préférence sous forme de ferrites : magnétite ou maghémite ou tout autre matériau inorganique ferro, ferri, méta ou antiferromagnétiques. Elles sont constituées de préférence de ferrite (Fe₂O₃) ou de magnétite. Elles peuvent être sous la forme d'un composite organo-minéral. Le minéral magnétique constituant le noyau de la particule est entouré d'une couche d'un composé organique. Les particules métalliques peuvent être obtenues par synthèse hydrothermale dans un réacteur agité en injectant 80 ml d'une solution de FeCL₂ calculée pour pouvoir synthétiser 5 g de magnétite, on ajoute ensuite 170 cm3 d'eau desaérée contenant 10 gr de NaOH. Sous débit d'azote (301/h) la solution est portée à 80°C. Lorsque cette température est atteinte, l'azote est remplacé par de l'air comprimé au même débit pendant 20 heures. Les ferrites sont ensuite lavés à l'eau puis à l'éthanol avant d'être séchés.

De préférence, les particules magnétiques ont une granulométrie comprise entre 0,001 µm et 0,01 µm, ou encore entre 0,05 µm et 0,1 µm, par exemple 0,07 µm, 0,15 µm, 0,5 µm. Elles peuvent, cependant, avoir une taille de l'ordre du micron pour des applications particulières, par exemple une granulométrie comprise entre 0,1 et 10 µm.

Un tel matériau forme ainsi une matrice minérale libérant les particules magnétiques suivant une cinétique compatible avec leur internalisation par les cellules des tissus avoisinants. Le matériau de l'invention peut résider en une association à une matrice minérale ou organique de particules magnétiques ou de particules de fer revêtues d'une couche de minéral préférentiellement de phosphate, sulfate ou carbonate de calcium. Ce revêtement peut également contenir un élément fluorescent tel que l'Europium. Plus spécifiquement, lesdites particules sont constituées d'un composite organo-minéral contenant un noyau de fer, de ferrite ou tout autre composé magnétique recouvert de polymère sous forme d'une couche mince ou sous forme de chaînes polymériques présentant un bout libre. Avantageusement, lesdites particules magnétiques sont vectrices d'une molécule utilisée en chimiothérapie ou bien un isotope.

Dans un deuxième aspect, l'invention porte sur un procédé de préparation dudit matériau comprenant le mélange d'une poudre de particules magnétiques à une poudre minérale de phosphate ou un sulfate de calcium en solution aqueuse jusqu'à formation d'une pâte, et en un durcissement de ladite pâte pendant quelques minutes à quelques heures. Ce procédé peut comprendre en outre une étape de préparation desdites particules par synthèse hydrothermale dans un réacteur par injection d'une solution de FeCL₂ dans un réacteur, addition d'eau desaérée contenant du NaOH, le mélange étant mis sous débit d'azote et portée à une température comprise en 50°C et 100°C, remplacement de l'azote par de l'air comprimé jusqu'à l'obtention de ferrites.

Les particules magnétiques une fois à l'intérieur des cellules sont destinées à être chauffées dans un champ magnétique pouvant être produit par exemple par un appareil d'imagerie par résonance magnétique nucléaire ou par tout autre générateur. Ainsi, dans un troisième aspect, l'invention concerne l'utilisation d'un matériau décrit ci-dessus pour la préparation d'un médicament ou d'un dispositif médical destiné au traitement des tumeurs osseuses. Plus particulièrement, ce dispositif médical permet la thermolyse ciblée des cellules cancéreuses. Ceci est rendu possible au moyen des particules magnétiques qui sont capables d'induire une hyperthermie dans les tissus dans lesquels ils sont libérés.

Un des avantages de cette méthode de chauffage dans un champ électromagnétique est de pouvoir, une fois l'injection faîte, être répétée le nombre de fois nécessaires sans nécessiter une autre injection. Les particules magnétiques sont, en effet, libérées sur plusieurs jours à plusieurs semaines à partir de la matrice phosphocalcique. Lorsque les cellules sont lysées, les particules qu'elles contenaient s'ajoutent à celles qui viennent d'être libérées de la matrice calcique pour être phagocytées par de nouvelles cellules. D'autre part, ces particules agissant au coeur de la tumeur n'entravent pas la régénération osseuse existant dans la périphérie des métastases osseuses.

Un autre avantage est de pouvoir être combinée aux autres méthodes de traitement actuellement connues, notamment la radiothérapie et/ou la chimiothérapie.

Dans un quatrième aspect, l'invention porte sur une méthode de diagnostic d'extension des cancers osseux comprenant l'utilisation des particules magnétiques comme traceurs de cellules tumorales détectables en IRM et le suivi des cellules en migration pour pouvoir traiter des sites à des stades infracliniques. Il faut souligner que pour le traçage cellulaire, les particules d'oxyde de fer de taille plus importante (~ 0,5 µm) produisent un signal de plus forte intensité (Hinds, K.A., et al. Highly efficient endosomal labelling of progenitor and stem cells with large magnetic particles allows magnetic resonance imaging of single cells. Blood, 2003).

L'invention porte également sur une méthode permettant le traçage en IRM, en microscopie électronique, focale ou de fluorescence des cellules ayant ingérées lesdites particules après relargage à partir d'un matériau dégradable et biocompatible décrit ci-dessus.

### Exemple 1 : Caractéristiques des particules magnétiques :

Obtention par synthèse hydrothermale
Composition Fe₂O₃
Forme octogonale
Propriétés magnétiques : H_{c} = 350 Oe, σᵣ= 32 uem/g
Taille 50 à 1500 nm.

### Exemple 2 : Caractéristiques de la poudre de sulfate de calcium :

Composition CaSO₄.2H₂O : 96%
CaCO₃.MgCO₃ : 2, 1 %
Fe₂O₃.Al₂O₃ : 0,5%
SiO₂:0,4%
Taille moyenne des particules : 17 - 20µm
pH de la solution de gâchage à 10% : 7,6
Solubilité pour 100 ml d'eau : 0,3 g
Surface spécifique : 2,5

### Exemple 3 : Endocytose des particules magnétiques

1 mg de particules magnétiques précédemment décrites de 0,07 µm de granulométrie a été introduit dans une culture de lignée primaire de cellules métastatiques osseuses afin de vérifier les capacités de ces cellules à phagocyter les particules. La lignée cellulaire a été obtenue à partir d'une biopsie d'une métastase osseuse d'un adenocarcinome du sein lors d'une intervention pour ostéosynthèse. Les cellules contenues dans la biopsie ont été dissociées par incubation pendant deux heures à 37°C dans une solution de collagénase dans un tampon phosphate isotonique. La suspension cellulaire a ensuite été centrifugée, remise en suspension dans 2 ml de milieu de culture DMEM supplémenté en 5% de sérum de veau foetal et introduit dans un flacon de culture à une densité de 10⁵ cellules/ml. Les cellules ont été cultivées pendant trois jours jusqu'à un stade de pré-confluence. Elles forment alors un tapis laissant un espace entre les cellules dont certaines sont sphériques marquant ainsi une prolifération élevée. 1 mg de poudre est ensuite mis en suspension par agitation dans 1 ml de milieu de culture et 0,5 ml est introduit dans le flacon de culture dans lequel on le dilue par l'apport de 3 ml de milieu de culture. Les cellules ont ensuite incubées à 37°C pendant 48 heures. A l'issue de cette période de culture les cellules sont observées en microscopie optique inversée puis le fond de la boîte est raclé et les cellules recueillies sont déshydratées, incluses dans une résine epoxy. Des coupes de 250 amstrongs sont faîtes et observées en microscopie électronique à transmission. L'examen en microscopie optique révèle des particules de différentes tailles (agglomérat de particules) à l'intérieur du cytoplasme des cellules. Les coupes en microscopie électronique montrent que le cytoplasme contient de nombreuses vésicules de type lysosomiales remplies de une ou plusieurs particules métalliques. Entre 10 et 30% des surfaces de section des cellules sont occupées par des particules.

### Exemple 4 : Test des différentes granulométries

1 mg de particules magnétiques précédemment décrites a été introduit dans une culture de lignée primaire de la même lignée de cellules métastatiques osseuses afin de vérifier les capacités de ces cellules à phagocyter les particules. Les particules ont quatre granulométries différentes : 0,07 µm, 0,15 µm, 0,5 µm. La lignée cellulaire a été obtenue à partir d'une biopsie d'une métastase osseuse d'un adenocarcinome du sein. Les cellules contenues dans la biopsie ont été dissociées par incubation pendant deux heures à 37°C dans une solution de collagénase dans un tampon phosphate isotonique. La suspension cellulaire a ensuite été centrifugée, remise en suspension dans 2 ml de milieu de culture DMEM supplémenté en 5% de sérum de veau foetal et introduit dans un flacon de culture à une densité de 10⁵ cellules/ml. Les cellules ont été cultivées pendant trois jours jusqu'à un stade de pré-confluence. Elles forment alors un tapis laissant un espace entre les cellules dont certaines sont sphériques marquant ainsi une prolifération élevée. 1 mg de poudre de chaque échantillon est ensuite mis en suspension par agitation dans 1 ml de milieu de culture et 0,5 ml est introduit dans un flacon de culture dans lequel on le dilue par l'apport de 3 ml de milieu de culture. Chaque essai est répété trois fois. Les cellules ont incubées à 37°C pendant 48 heures. Les cultures sont ensuite examinées en microscopie optique inversée et en microscopie électronique par transmission.

Quelle que soit la granulométrie, de nombreuses particules sont en quelques heures au contact des cellules et à la fin du temps d'observation, elles ont pénétré dans les cellules. Comme cela a été rapporté par Hinds (Hinds, K.A., et al. Highly efficient endosomal labelling of progenitor and stem cells with large magnetic particles allows magnetic résonance imaging of single cells. Blood, 2003), l'internalisation des particules n'a pas semblé induire de mort cellulaire lors de la période de culture.

### Exemple 5 : Dégradation des matrices minérales in vivo

Quatre moutons adultes ont été anesthésiés et un trou de 4mm de diamétre et de 5 mm de long a été effectué dans le condyle externe droit. Le trou a ensuite été rempli avec une pâte constituée de sulfate de calcium qui a été laissé prendre *in situ.* Deux moutons ont été euthanasiés à deux semaines et les deux autres à quatre semaines. Les condyles ont été prélevés, déshydratés dans de l'éthanol et inclus dans des blocs de polyméthyl méthacrylate. Des coupes de 7 µm d'épaisseur ont été effectuées, colorées par une solution de Giemsa et observées en microscopie optique. A deux semaines, le plâtre injecté est toujours visible. Il est en voie de résorption, avec des encoches dans la matrice minérale. L'implant est entouré de tissu conjonctif lâche avec en périphérie immédiate de l'implant des monocytes et des cellules géantes. Il existe des signes d'ostéogénèse au niveau des trabécules osseux dans lequel le plâtre est implanté. Des fragments de matériaux sont visibles, et les cellules au contact du matériau selon l'invention contiennent des grains de minéral. A quatre semaines, l'implant a disparu. Il reste dans la zone d'implantation, une zone de tissu conjonctif lâche qui est beaucoup plus petite que la section de l'implant. Un tissu osseux immature poreux a pénétré dans la zone d'implantation. Il existe encore quelques fragments d'implants et les cellules macrophagiques contiennent encore quelques grains de matériau. Il n'existe aucun signe d'ostéolyse.

### Exemple 6 : Délivrance des particules magnétiques à des cellules par la matrice in vivo

Quatre moutons adultes ont été anesthésiés et un trou de 4mm de diamètre et de 5 mm de long du condyle externe droit a été effectué. Le trou a ensuite été rempli avec une pâte constituée de plâtre de Paris contenant 2 mg de particules magnétiques par ml de solution de gâchage. Deux moutons ont été euthanasiés à deux semaines et les deux autres à quatre semaines. Les condyles ont été prélevés, déshydratés dans de l'éthanol et inclus dans des blocs de polyméthyl méthacrylate. Des coupes de 7 µm d'épaisseur ont été effectuées, colorées par une solution de Giemsa et observées en microscopie optique. A deux semaines, il existe un début de résorption du matériau qui est entouré d'un tissu conjonctif lâche. Les cellules macrophagiques au contact du matériau contiennent des particules minérales biréfringentes ainsi que des particules magnétiques noires qui réalisent un tatouage du cytoplasme. Il faut noter que des cellules conjonctives qui n'ont pas les caractéristiques morphologiques de macrophages montrent également un cytoplasme tatoué. A un mois, l'implant a disparu et une grande partie de la surface de section occupée au préalable par l'implant est envahi par des trabécules osseux. L'espace intertrabéculaire est occupé par un tissu conjonctif lâche dont toutes les cellules sont tatouées par les particules magnétiques. L'étude en microscopie à transmission confirme que de nombreuses cellules autour de l'implant contiennent des particules métalliques.

## Revendications

1. Matériau composite dégradable biocompatible, **caractérisé en ce qu'**il est composé d'une matrice phosphocalcique et/ou de sulfate de calcium biocompatible dégradable, ladite matrice contenant des particules magnétiques, ledit matériau se trouvant sous la forme d'une pâte lors de l'introduction dans l'organisme, sous forme solide ultérieurement et ladite matrice étant résorbée dans un délai de quelques jours à quelques semaines.

2. Matériau composite selon la revendication 1, **caractérisé en ce que** le phosphate de calcium est un mélange comprenant un phosphate sélectionné parmi le groupe des phosphates de calcium amorphe, des phosphates apatitiques faiblement cristallins, des phosphates dicalciques anhydres ou dihydratés, des phosphates tricalciques, des phosphates monocalciques monohydratés, des pyrophosphates, des phosphates octocalciques ou de l'hydroxyapatite.

3. Matériau selon l'une des revendications 1 et 2, **caractérisé en ce que** ledit phosphate de calcium forme une matrice phosphocalcique rapidement résorbable.

4. Matériau selon l'une des revendications 1 à 3, comprenant en outre du sulfate de calcium.

5. Matériau selon l'une des revendications 1 à 4 **caractérisé en ce qu'**il est composé en outre d'une matrice polymérique biocompatible dégradable comprenant un polymère choisi parmi le collagène, les acides polylactiques et glycoliques, le polydioxanone, le polyfumarate, les polyanhydres, les polyorthoesters, les polyurethanes, les polyphosphazenes, le polycaprolactone, le polyhydroxybutyrate, le polyhydroxyvalerate, la polyvalerolactone, l'acide polytartronique et polymalonique; contenant des particules magnétiques.

6. Matériau selon l'une des revendications 1 à 4, **caractérisé en ce que** ladite matrice présente des caractéristiques de biocompatibilité et de dégradation compatibles avec les applications du matériau pour le traitement des tumeurs osseuses.

7. Matériau selon l'une des revendications 1 à 5, **caractérisé en ce que** les particules magnétiques contiennent un métal, notamment du fer, de préférence sous forme de ferrites : magnétite ou maghémite ou tout autre matériau inorganique ferro, ferri, méta ou antiferromagnétiques.

8. Matériau selon l'une des revendications 1 à 6, **caractérisé en ce que** lesdites particules sont constituées d'un composite organo-minéral contenant un noyau de fer, de ferrite ou tout autre composé magnétique recouvert de polymère sous forme d'une couche mince ou sous forme de chaînes polymériques présentant un bout libre

9. Matériau selon l'une des revendications 1 à 8, **caractérisé en ce que** les dites particules magnétiques sont vectrices d'une molécule utilisée en chimiothérapie ou bien un isotope.

10. Matériau selon l'une des revendications 1 à 9, **caractérisé en ce que** lesdites particules ont une granulométrie comprise entre 0,001 et 0,1 µm.

11. Matériau selon l'une des revendications 1 à 9, **caractérisé en ce que** lesdites particules ont une granulométrie comprise entre 0,1 et 10 µm

12. Matériau selon l'une des revendications 1 à 11 formant une matrice minérale libérant les particules magnétiques suivant une cinétique compatible avec leur internalisation par les cellules des tissus avoisinants.

13. Matériau selon l'une des revendications 1 à 11, **caractérisé en ce qu'**il comprend des particules revêtues d'une couche de phosphate de calcium contenant un élément fluorescent tel que l'Europium.

14. Procédé de préparation d'un matériau selon l'une des revendications 1 à 10 comprenant le mélange d'une poudre de particule magnétique à une poudre minérale de phosphate ou de sulfate de calcium-en solution aqueuse jusqu'à formation d'une pâte, et en un durcissement de ladite pâte pendant quelques minutes à quelques heures.

15. Procédé de préparation d'un matériau selon la revendication 10 comprenant en outre une étape de préparation desdites particules par synthèse hydrothermale dans un réacteur par injection d'une solution de FeCL₂ dans un réacteur, addition d'eau desaérée contenant du NaOH, le mélange étant mis sous débit d'azote et porté à une température comprise en 50°C et 100°C, remplacement de l'azote par de l'air comprimé jusqu'à l'obtention de ferrites.

16. Utilisation d'un matériau selon l'une des revendications 1 à 13 pour la préparation d'un dispositif pour le diagnostic des cancers osseux comprenant l'utilisation des particules magnétiques contenues dans ledit matériau comme traceurs de cellules tumorales détectables en IRM et le suivi des cellules en migration pour pouvoir traiter des sites à des stades infracliniques.

17. Utilisation d'un matériau selon l'une des revendications 1 à 13 pour la préparation d'un dispositif pour le traçage des cellules ayant ingéré les particules après relargage à partir dudit matériau dégradable et biocompatible au moyen d'IRM, microscopie électronique, microscopie confocale ou miscroscopie en fluorescence.

18. Utilisation d'un matériau selon l'une des revendications 1 à 10 pour la préparation d'un médicament ou un dispositif médical destiné au traitement des tumeurs osseuses.

19. Utilisation selon la revendication 18 pour la thermolyse ciblée des cellules cancéreuses.

20. Utilisation selon la revendication 19 **caractérisé en ce que** les particules magnétiques une fois à l'intérieur des cellules sont destinées à être chauffées dans un champ magnétique pouvant être produit par un appareil d'imagerie par résonance magnétique nucléaire ou par tout autre générateur.

21. Utilisation selon l'une des revendications 18 à 20 combinée avec la radiothérapie et/ou la chimiothérapie.

## Claims

1. A biocompatible degradable composite material, **characterized in that** it consists of a degradable biocompatible phosphocalcium and/or calcium sulfate matrix, said matrix containing magnetic particles, said material being found as a slurry during its introduction into the organism, as a solid subsequently and said matrix being resorbed within a period of a few days to a few weeks.

2. The composite material according to claim 1, **characterized in that** the calcium phosphate is a mixture comprising a phosphate selected from the group of amorphous calcium phosphates, low crystalline apatite phosphates, anhydrous dicalcium phosphates or dicalcium phosphate dehydrates, tricalcium phosphates, monocalcium phosphate monohydrates, pyrophosphates, octocalcium phosphates, or hydroxyapatite.

3. The material according to any of claims 1 and 2, **characterized in that** said calcium phosphate forms a rapidly resorbable phosphocalcium matrix.

4. The material according to any of claims 1 to 3, further comprising calcium sulfate.

5. The material according to any of claims 1 to 4, **characterized in that** it further consists of a degradable biocompatible polymer matrix comprising a polymer selected from collagen, polylactic and glycolic acids, polydioxanone, polyfumarate, polyanhydrides, polyorthoesters, polyurethanes, polyphosphazenes, polycaprolactone, polyhydroxybutyrate, polyhydroxy-valerate, polyvalerolactone, polytartronic and polymalonic acid; containing magnetic particles.

6. The material according to any of claims 1 to 4, **characterized in that** said matrix has biocompatibility and degradation characteristics compatible with applications of the material for treating bone tumors.

7. The material according to any of claims 1 to 5, **characterized in that** the magnetic particles contain a metal, notably iron, preferably as ferrites: magnetite or maghemite or any other ferro-, ferri-magnetic, meta- or antiferromagnetic inorganic material.

8. The material according to any of claims 1 to 6, **characterized in that** said particles consist of an organomineral composite containing an iron, ferrite core, or core of any other magnetic compound coated with polymer as a thin layer or as polymeric chains having a free end.

9. The material according to any of claims 1 to 8, **characterized in that** said magnetic particles are vectors either of a molecule used in chemotherapy or an isotope.

10. The material according to any of claims 1 to 9, **characterized in that** said particles have a particle size between 0.001 and 0.1 *µ*m.

11. The material according to any of claims 1 to 9, **characterized in that** said particles have a particle size between 0.1 and 10 µm.

12. The material according to any of claims 1 to 11, forming a mineral matrix releasing magnetic particles according to kinetics compatible with their internalization by cells from neighboring tissues.

13. The material according to any of claims 1 to 11, **characterized in that** it comprises particles coated with a calcium phosphate layer containing a fluorescent element such as europium.

14. A method for preparing a material according to any of claims 1 to 10, comprising mixing of a magnetic particle powder with a calcium sulfate or phosphate mineral powder, in an aqueous solution until a slurry is formed, and hardening said slurry for a few minutes to a few hours.

15. The method for preparing a material according to claim 10, further comprising a step for preparing said particles by hydrothermal synthesis in a reactor by injecting a FeCl₂ solution, adding deaerated water containing NaOH, the mixture being placed under nitrogen flow and brought to a temperature between 50°C and 100°C, replacing nitrogen with compressed air until ferrites are obtained.

16. Use of a material according to any of claims 1 to 13 for preparing a device for diagnosing bone cancers comprising the use of magnetic particles contained in said materials as tracers of MRI-detectable tumor cells and the tracking of the migrating cells in order to be able to treat sites at infraclinic stages.

17. Use of a material according to any of claims 1 to 13 for preparing a device for tracing cells having ingested particles after desalting from said degradable and biocompatible material by means of MRI, electronic microscopy, confocal microscopy, or fluorescence microscopy.

18. Use of a material according to any of claims 1 to 10 for preparing a drug or a medical device for treating bone tumors.

19. Use according to claim 18 for targeted thermolysis of cancer cells.

20. Use according to claim 19, **characterized in that** the magnetic particles once inside the cells are intended to be heated in a magnetic field which may produced by a nuclear magnetic resonance imaging apparatus or any other generator.

21. Use according to any of claims 18 to 20, combined with radiotherapy and/or chemotherapy.

## Patentansprüche

1. Biologisch verträgliches, abbaubares Verbundmaterial, **dadurch gekennzeichnet, dass** es eine biologisch verträgliche, abbaubare Calciumphosphat- und/oder Calciumsulfat-Matrix umfasst, wobei die Matrix magnetische Teilchen enthält, wobei das Material während des Einführens in den Organismus in Form einer Paste und später in fester Form vorliegt und wobei die Matrix mit einer Verzögerung von einigen Tagen bis einigen Wochen resorbiert wird.

2. Verbundmaterial nach Anspruch 1, **dadurch gekennzeichnet, dass** das Calciumphosphat eine Mischung ist, welche ein Phosphat, ausgewählt aus der Gruppe der amorphen Calciumphosphate, der geringfügig kristallinen apatitischen Phosphate, der wasserfreien oder in Dihydrat-Form vorliegenden Dicalciumphosphate, der Tricalciumphosphate, der in Monohydrat-Form vorliegenden Monocalciumphosphate, der Pyrophosphate, der Octocalciumphosphate oder von Hydroxylapatit, umfasst.

3. Material nach einem der Ansprüche 1 und 2, **dadurch gekennzeichnet, dass** das Calciumphosphat eine schnell resorbierbare Calciumphosphat-Matrix bildet.

4. Material nach einem der Ansprüche 1 bis 3, welches außerdem Calciumsulfat umfasst.

5. Material nach einem der Ansprüche 1 bis 4, **dadurch gekennzeichnet, dass** es außerdem eine biologisch verträgliche, abbaubare, polymere Matrix, umfassend ein Polymer, ausgewählt unter Collagen, den Polymilch- und Glycolsäuren, Polydioxanon, Polyfumarat, den Polyanhydriden, den Polyorthoestern, den Polyurethanen, den Polyphosphazenen, Polycaprolacton, Polyhydroxybutyrat, Polyhydroxyvalerat, Polyvalerolacton, Polytartron- und Polymalonsäure, welche magnetische Teilchen enthält, umfasst.

6. Material nach einem der Ansprüche 1 bis 4, **dadurch gekennzeichnet, dass** die Matrix Merkmale von biologischer Verträglichkeit und Abbaumerkmale, die mit den Anwendungen des Materials für die Behandlung von Knochentumoren verträglich sind, aufweist.

7. Material nach einem der Ansprüche 1 bis 5, **dadurch gekennzeichnet, dass** die magnetischen Teilchen ein Metall, insbesondere Eisen, vorzugsweise in Form von Ferriten: Magnetit oder Maghemit oder ein jegliches anderes anorganisches Eisen(II)-, Eisen(III)-, meta- oder antiferromagnetisches Material enthalten.

8. Material nach einem der Ansprüche 1 bis 6, **dadurch gekennzeichnet, dass** die Teilchen ein organisch-anorganisches Verbundmaterial, enthaltend einen Kern von Eisen, von Ferrit oder einer jeglichen anderen magnetischen Verbindung, überzogen von Polymer in Form einer dünnen Schicht oder in Form von Polymerketten, welche ein freies Ende aufweisen, umfassen.

9. Material nach einem der Ansprüche 1 bis 8, **dadurch gekennzeichnet, dass** die magnetischen Teilchen Träger für ein Molekül, das in der Chemotherapie eingesetzt wird, oder ebenso ein Isotop sind.

10. Material nach einem der Ansprüche 1 bis 9, **dadurch gekennzeichnet, dass** die Teilchen eine Korngröße (Granulometrie) zwischen 0,001 und 0,1 µm eingeschlossen aufweisen.

11. Material nach einem der Ansprüche 1 bis 9, **dadurch gekennzeichnet, dass** die Teilchen eine Korngröße (Granulometrie) zwischen 0,1 und 10 µm eingeschlossen aufweisen.

12. Material nach einem der Ansprüche 1 bis 11, welches eine anorganische Matrix bildet, welche die magnetischen Teilchen entsprechend einer Kinetik, welche mit deren Internalisierung durch die Zellen der angrenzenden Gewebe verträglich ist, freisetzt.

13. Material nach einem der Ansprüche 1 bis 11, **dadurch gekennzeichnet, dass** es Teilchen umfasst, die von einer Calciumphosphat-Schicht, welche ein fluoreszierendes Element, wie Europium, enthält, überzogen sind.

14. Verfahren zur Herstellung eines Materials nach einem der Ansprüche 1 bis 10, welches das Mischen eines Pulvers von magnetischen Teilchen mit einem anorganischen Pulver von Calciumphosphat oder -sulfat in wässriger Lösung bis zur Bildung einer Paste und ein Härten dieser Paste während einiger Minuten bis einiger Stunden umfasst.

15. Verfahren zur Herstellung eines Materials nach Anspruch 10, welches außerdem einen Schritt einer Herstellung der Teilchen durch hydrothermale Synthese in einem Reaktor durch Einspritzen einer Lösung von FeCl₂ in einen Reaktor, Hinzufügen von entlüftetem Wasser, welches NaOH enthält, wobei die Mischung unter Stickstoffzufuhr gesetzt und auf eine Temperatur zwischen 50°C und 100°C erwärmt wird, Ersetzen des Stickstoffs durch Druckluft bis zur Gewinnung von Ferriten umfasst.

16. Verwendung eines Materials nach einem der Ansprüche 1 bis 13 für die Herstellung einer Vorrichtung für die Diagnostik von Knochenkrebserkrankungen, umfassend die Verwendung der magnetischen Teilchen, welche in dem Material enthalten sind, als Tracer von Tumorzellen, die mittels MRI detektiert werden können, und das Verfolgen der in Migration befindlichen Zellen, um Stellen in infraklinischen Stadien behandeln zu können.

17. Verwendung eines Materials nach einem der Ansprüche 1 bis 13 für die Herstellung einer Vorrichtung für das Verfolgen der Zellen, die die Teilchen nach Freisetzung aus dem abbaubaren und biologisch verträglichen Material aufgenommen haben, mittels MRI, Elektronenmikroskopie, konfokale Mikroskopie oder Fluoreszenzmikroskopie.

18. Verwendung eines Materials nach einem der Ansprüche 1 bis 10 für die Herstellung eines Arzneimittels oder einer medizinischen Vorrichtung, welche(s) für die Behandlung von Knochentumoren bestimmt ist.

19. Verwendung nach Anspruch 18 für die zielgerichtete Thermolyse von Krebszellen.

20. Verwendung nach Anspruch 19, **dadurch gekennzeichnet, dass** die magnetischen Teilchen, sind sie einmal im Inneren der Zellen, dazu bestimmt sind, erwärmt zu werden in einem Magnetfeld, welches durch eine Bildgebungsvorrichtung durch Kernspinresonanz oder durch eine jegliche andere Erzeugungsvorrichtung erzeugt werden kann.

21. Verwendung nach einem der Ansprüche 18 bis 20 kombiniert mit Strahlentherapie und/oder Chemotherapie.
